(19) Europäisches Patentamt
European Patent Office
Office européen des brevets

(11) Publication number: **0 026 816**
**B1**

(12) # EUROPEAN PATENT SPECIFICATION

(45) Date of publication of patent specification: **30.05.84**

(51) Int. Cl.$^3$: **C 07 D 205/08**, C 07 F 7/10 // C07D487/04

(21) Application number: **80104296.1**

(22) Date of filing: **22.07.80**

(54) **Intermediates for the preparation of thienamycin and process for preparing intermediates.**

(30) Priority: **23.07.79 US 59844**

(43) Date of publication of application:
**15.04.81 Bulletin 81/15**

(45) Publication of the grant of the patent:
**30.05.84 Bulletin 84/22**

(84) Designated Contracting States:
**AT BE CH DE FR GB IT LI LU NL SE**

(56) References cited:
**EP-A-0 007 973**

**Journal of the American Chemical Society, 100 (1978), p. 313-315**

(73) Proprietor: **MERCK & CO. INC.**
**126, East Lincoln Avenue P.O. Box 2000**
**Rahway New Jersey 07065 (US)**

(72) Inventor: **Christensen, Burton G.**
**195 Watchung Terrace**
**Scotch Plains, N.J. 07076 (US)**
Inventor: **Ratcliffe, Ronald W.**
**234 Matawan Avenue**
**Matawan, N.J. 07747 (US)**
Inventor: **Salzmann, Thomas N.**
**387 Brook Avenue**
**North Plainfiels, N.J. 07062 (US)**

(74) Representative: **Abitz, Walter, Dr.-Ing. et al**
**Abitz, Morf, Gritschneder P.O. Box 86 01 09**
**D-8000 München 86 (DE)**

**EP 0 026 816 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

A total synthesis of the antibiotic thienamycin (I)

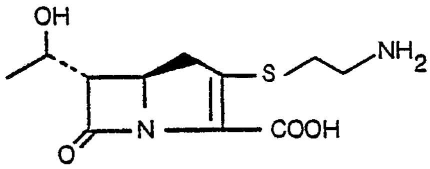

I

is known from J. Am. Chem. Soc. 100 (1978), 313—315.

A preparation of thienamycin and intermediates is also described in EP—A—0 007 973 which has been published after the priority date of the present invention.

Detailed Description of the Invention

The invention relates to intermediates for a total synthesis of thienamycin.

The intermediates of the invention are compounds selected from the group consisting of:

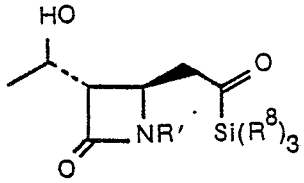

23

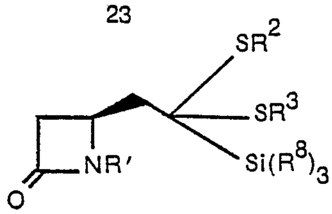

21

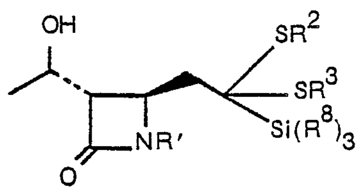

22

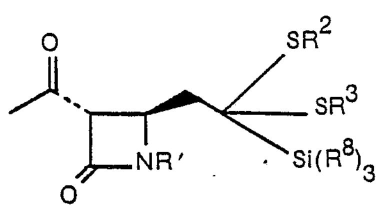

22a

wherein $R^2$ and $R^3$ are independently selected from: lower alkyl having 1—6 carbon atoms, phenyl, benzyl, methoxybenzyl, or trityl; R′ is a triorgano silyl group, 3,4-dimethoxybenzyl or hydrogen; $R^8$ is lower alkyl having 1—6 carbon atoms, or phenyl, or $R^2$ and $R^3$ together form $-(CH_2)_3-$. Preferred are compounds, wherein:

R′ is triloweralkyl silyl having from 3—12 carbon atoms;

$R^8$ is alkyl having 1—3 carbon atoms, or phenyl; and

$R^2$ and $R^3$ are: $-(CH_2)_3-$ or alkyl having 1—3 carbon atoms each.

The invention also relates to a process for preparing

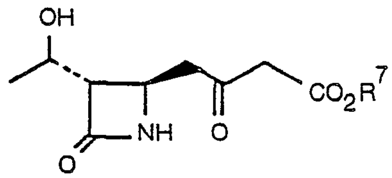

16

comprising:
oxidizing:

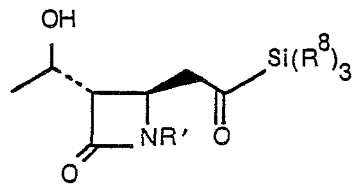

23

to form:

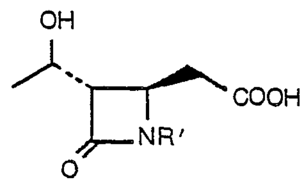

24

followed by treating with $R^7O_2CCH_2CO_2^{\ominus}$ and preferably $(R^7O_2CCH_2CO_2)_2Mg$ to yield:

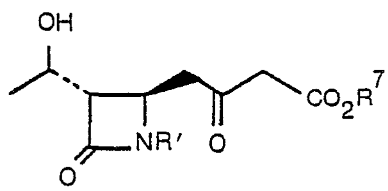

25

wherein $R^7$ is a carboxyl protecting group and R′ is a triorgano silyl group or 3,4-dimethoxybenzyl and removal of the protecting group R′. $R^7$ is typically an aralkyl group such as p-nitrobenzyl, or o-nitrobenzyl.

Further the invention relates to a compound having the structure:

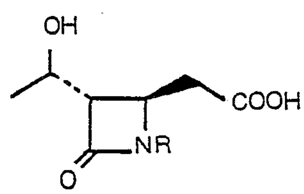

24

wherein R is a triorgano silyl group or 3,4-dimethoxybenzyl. Preferably R is trialkyl silyl wherein the alkyl moieties have 1—6 carbon atoms each, and most preferable R is (*t*-butyl)dimethylsilyl.

The process of the present invention may conveniently be illustrated by the following reaction diagram, which includes preparation of starting compounds and further processing of the intermediates of the invention, to prepare thienamycin.

A) Preparation of starting compounds:

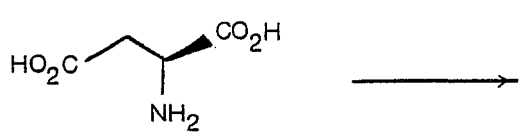

1

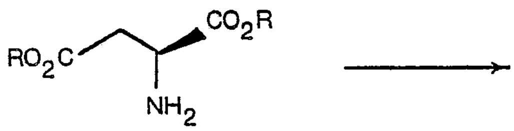

2

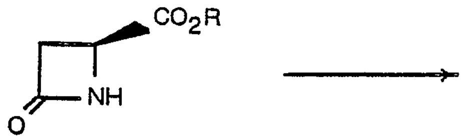

3

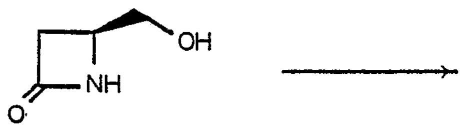

4

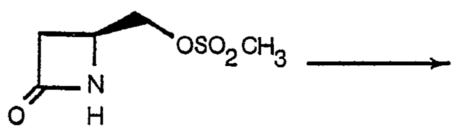

4a

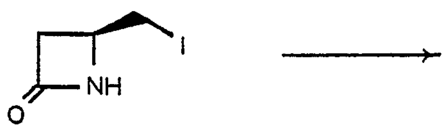

5

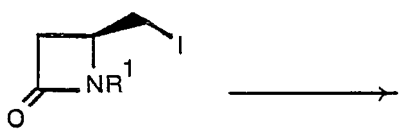

6

B) Preparation of the intermediates of the invention:

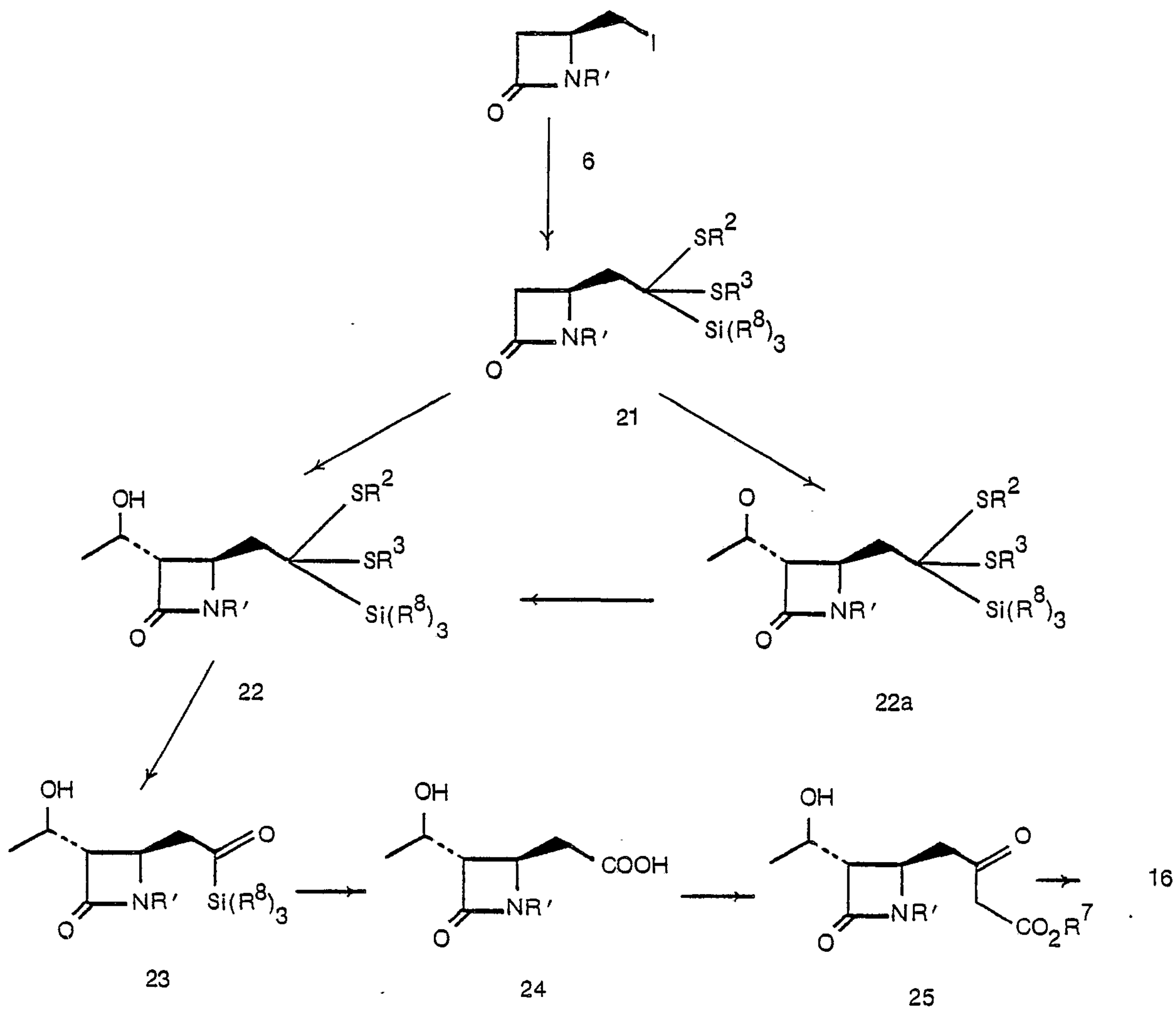

6

21

22

22a

23

24

25

16

C) Further processing of the intermediates of the invention, to prepare thienamycin:

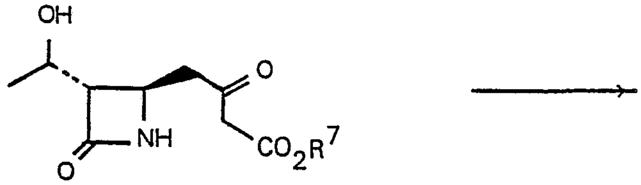

16

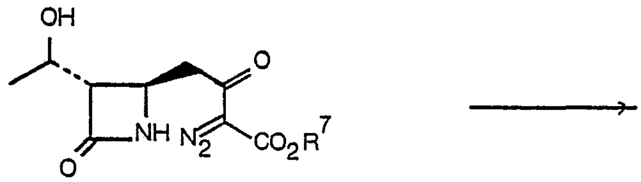

17

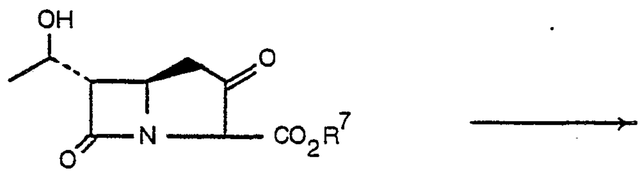

18

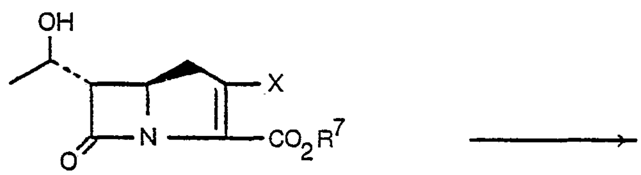

19

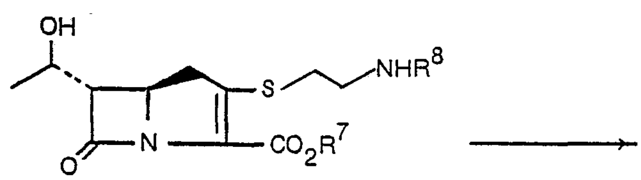

20

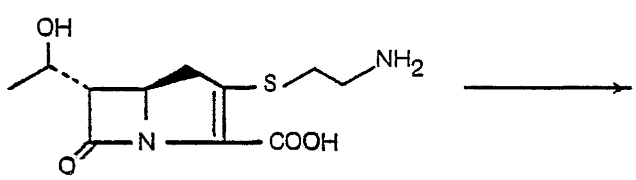

I

In words relative to the above diagram, for preparing the starting compounds, L-aspartic acid 1 is esterified according to well known procedures. Typically 1 in a solvent such as benzene, toluene or chloroform is treated with an esterifying agent such as benzyl alcohol, methanol, ethanol or isopropanol, in the presence of p-toluene sulfonic acid, HCl or HBr, at a temperature of from 0 to 110°C for from 1 to 24 hours to achieve the desired establishment and hence protection of the carboxyl functions. The resulting species 2 in a solvent such as ether, THF or DME is treated with trimethylchlorosilane, for example, followed by treatment with EtMgBr, MeMgI, ØMgBr, t-BuMgCl, for example, at a temperature of from −40 to 50°C for from 1 to 72 hours to provide azetidinone 3. Reduction of species 3 with a reducing agent such as $NaBH_4$, in a solvent such as methanol, ethanol or isopropanol at a temperature of from −10 to 40°C for from 1 to 6 hours provides 4. (For purposes here, the symbols: Et, Me, Ø, iPr, and t-Bu stand for: ethyl, methyl, phenyl, isopropyl, and tert-butyl, respectively).

Treatment of 4 in a solvent such as methylene chloride or $CHCl_3$ with methane sulfonyl chloride, methane sulfonic anhydride, for example, in the presence of a base such as $Et_3N$ or $iPr_2NEt$, followed by treatment with a stoichiometric to 5 fold excess of sodium iodide in acetone yields 5 via 4a.

The transformation 5 → 6 establishes the protecting group $R^1$ which is a triorganosilyl group, such as t-butyldimethylsilyl, t-butyldiphenylsilyl, triphenylsilyl, isopropyldimethylsilyl, for example, or may be 3,4-dimethoxybenzyl. Silyl protection is preferred, and typically $R^1$ is established by treating 5 in a solvent such as dimethylformamide, acetonitrile, hexamethylphosphoramide or tetrahydrofuran with a silylating agent such as t-butyldimethylchlorosilane, t-butyldiphenylchlorosilane or triphenylchlorosilane, at a temperature of from −20° to 25°C for from 0.5 to 24 hours in the present of a base such as triethylamine, diisopropylethylamine, or imidazole.

The transformation 6 → 21 is accomplished by treating 6 in a solvent such as tetrahydrofuran, dimethoxyethane or diethylether with a carbanion generically represented by the following structure:

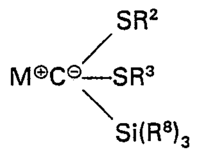

wherein M is a metal cation such as lithium, potassium, copper or magnesium, and $R^2$, $R^3$ and $R^8$ are defined as above (wherein alkyl is preferably methyl or ethyl), at a temperature of from −100 to 0°C and from 0.5 to 4 hours.

Typically, the carbanion reagent is prepared prior to addition of substrate 6 on treatment of the triorganothiomethane with a strong base such as n-butyllithium, t-butyllithium, phenyllithium or lithium diisopropylamide (LDA).

The alkylation 21 → 22 is accomplished by treating 21 in a solvent such as tetrahydrofuran, dimethoxyethane, diethylether, hexamethylphosphoramide, at a temperature of from −100° to −20°C with a strong base such as lithium diisopropylamide, lithium hexamethyldisilazide, lithium 2,2,6,6-tetramethylpiperidide or potassium hydride followed by the addition of an acylating agent such as N-acetyl imidazole or an equivalent to 10 fold excess of acetaldehyde. This aldol reaction gives a mixture of isomers from which the desired *trans*-R form can be conveniently separated by chromatography or crystallization. An indirect aldol reaction scheme stereo-selectively provides the desired trans-R isomer according to the circuitous path via 22a. The transformation 21 → 22a → 22 is conducted via an oxidation step with an oxidizing agent such as dipyridine chromium (VI)oxide, trifluoroacetic anhydride-dimethylsulfoxide-triethylamine, pyridinium dichromate, acetic anhydride-dimethylsulfoxide in a solvent such as methylene chloride or acetonitrile, at a temperature of from −78 to 25°C for from 5 minutes to 5 hours.

The following step is accomplished by contacting the ketone with a reducing agent such as potassium tri(sec-butyl)borohydride, lithium tri(sec-butyl)borohydride, sodium borohydride, sodium tris(methoxyethoxy)aluminum hydride or lithium aluminum hydride in a solvent such as diethylether, tetrahydrofuran or toluene at a temperature of from −20 to 25°C. The reaction can conveniently be conducted in the presence of an added complexing salt such as potassium iodide or magnesium bromide.

The transformation 22 → 23 is accomplished by treating 22 in a solvent such as methanol, ethanol, isopropanol or water at a temperature of from 0 to 80°C with a Lewis acid such as mercuric chloride, silver tetrafluoroborate or thallium trinitrate.

The oxidation 23 → 24 is preferably achieved with a 1.0 to 5.0 fold excess of an oxidizing agent such as m-chloroperbenzoic acid, peracetic acid, hydrogen peroxide or pertrifluoro acetic acid, in a solvent such as chloroform, carbontetrachloride or chlorobenzene, at a temperature of from 25°C to 130°C for from 0.5 to 24 hours.

The addition 24 → 25 is accomplished by treating 24 with 1,1′-carbonyldimidazole, for example, in a solvent such as tetrahydrofurane or dimethoxyethane, at a temperature of from 10° to 50°C followed by the addition of 1.1 to 3.0 equivalents of $(R^7O_2CCH_2CO_2)_2Mg$, for example, at a temperature of from −10° to 50°C for from 1 to 48 hours.

The deblocking of 25 to yield 16 is accomplished by acidic aqueous hydrolysis of 25 in a solvent such as methanol, ethanol, tetrahydrofuran or dioxane in the presence of an acid such as hydrochloric, sulfuric or acetic at a temperature of from 0 to 100°C for from 2 to 18 hours. The remainder of the synthesis as described in the above diagram, steps from 16 to thienamycin, is described in EP—A—0 007 973.

The diazo species 17 is prepared from 16 by treating 16 in a solvent such as $CH_3CN$, $CH_2Cl_2$ or THF with an azide such as p-carboxybenzene-sulfonylazide, toluenesulfonylazide or methanesulfonylazide in the presence of a base such as triethylamine, pyridine or $(C_2H_5)_2NH$ for from 1 to 50 hours at 0—25°C.

Cyclization (17 → 18) is accomplished by treating 17 in a solvent such as benzene, toluene or THF at a temperature of from 50—110°C for from 1—5 hours in the presence of a catalyst such as *bis* (acetylacetonato)Cu (II) [Cu(acac)$_2$], $CuSO_4$, Cu powder, $Rh(OAc)_2$, or $Pd(OAc)_2$. Alternatively, the cyclization may be accomplished by irradiating 17 through a pyrex filter (a wave length greater than 300 nm) in a solvent such as benzene, $CCl_4$ or diethylether at a temperature of from 0—25°C for from 0.5 to 2 hours. ["OAc" = acetate.]

Establishment of leaving group X (18 → 19) is accomplished by acylating the keto ester 18 with an acylating agent R°X such as p-toluenesulfonic acid anhydride, p-nitrophenylsulfonic acid anhydride, 2,4,6-triisopropylphenylsulfonic acid anhydride, methanesulfonic acid anhydride, toluenesulfonyl chloride or p-bromophenylsulfonyl chloride, wherein X is the corresponding leaving group such as toluene sulfonyloxy, p-nitrophenylsulfonyloxy, methanesulfonyloxy, p-bromophenylsulfonyloxy and other leaving groups which are established by conventional procedures and are well known in the art. Typically, the above acylation to establish leaving groups X is conducted in a solvent such as methylene chloride, acetonitrile or dimethylformamide, in the presence of a base such as diisopropylethylamine, triethylamine or 4-dimethylamino-pyridine at a temperature of from —20 to 40°C for from 0.5 to 5 hours. The leaving group X of intermediate 19 can also be halogen. The halogen leaving group is established by treating 18 with a halogenating agent such as $Ø_3PCl_2$, $Ø_3PBr_2$, $(ØO)_3PBr_2$ or oxalyl chloride in a solvent such as $CH_2Cl_2$, $CH_3CN$ or THF, in the presence of a base such as diisopropylethylamine, triethylamine or 4-dimethylaminopyridine. [Ø = phenyl.]

The reaction 19 → 20 is accomplished by treating 19 in a solvent such as dioxane, dimethylformamide, dimethylsulfoxide, acetonitrile or hexamethylphosphoramide, in the presence of an approximately equivalent to excess of the mercaptan reagent $HSCH_2CH_2NHR^8$ wherein $R^8$ is hydrogen or a readily removable N-protecting group such as p-nitrobenzyloxycarbonyl or o-nitrobenzyloxycarbonyl, in the presence of a base such as sodium hydrogen carbonate, potassium carbonate, triethylamine or diisopropylethylamine, at a temperature of from —40 to 25°C for from 1 to 72 hours. The mercaptan reagent, $HSCH_2CH_2NHR^8$, is typically prepared by treating aminoethylmercaptan in the presence of the desired acid chloride in the presence of a base such as sodium bicarbonate or sodium hydroxide, in a solvent such as aqueous diethylether, aqueous dioxane or aqueous acetone, at a temperature of from 0 to 25°C for from 0.5 to 4 hours.

The final deblocking step 20 → I is accomplished by conventional procedures such as hydrolysis or hydrogenation. Typically 20 in a solvent such as dioxane-water-ethanol or tetrahydrofuran-aqueous dipotassium hydrogen phosphate-isopropanol is treated under a hydrogen pressure of from 1 to 4 atmospheres in the presence of a hydrogenation catalyst such as palladium on charcoal or palladium hydroxide, at a temperature of from 0 to 50°C for from 0.5 to 4 hours to provide I.

In the foregoing word description of the above schematic reaction diagram for the total synthesis of thienamycin, it is to be understood that there is considerable latitude in selection of precise reaction parameters. Suggestion of this latitude and its breadth is generally indicated by the enumeration of equivalent solvent systems, temperature ranges, protecting groups, and range of identities of involved reagents. Further, it is, to be understood that the presentation of the synthetic scheme as comprising distinct steps in a given sequence is more in the nature of a descriptive convenience than as a necessary requirement; for one will recognize that the mechanically dissected scheme represents a unified scheme of synthesis and that certain steps, in actual practice, are capable of being merged, conducted simultaneously, or effected in a reverse sequence without materially altering the progress of synthesis.

The following examples recite a precise scheme of total synthesis to further illustrate the total synthesis.

Example 1

Preparation of (4S)-1-(t-butyldimethylsilyl)-4-[2,2-(trimethylene-dithia)-2-trimethylsilylethyl]-azetidin-2-one (21).

*Step A*

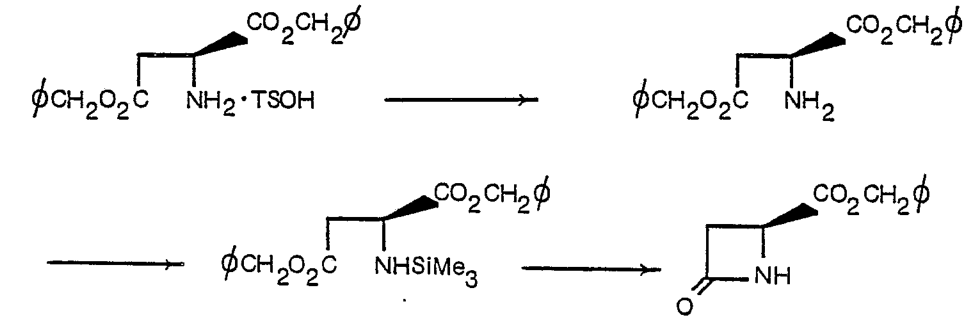

Benzyl (*S*)-azetidin-2-one-4-carboxylate

To a 1000 ml separatory funnel are added dibenzyl (*S*)-aspartate *p*-toluenesulfonic acid salt (48.6 g, 0.1 mole), ice-cold diethyl ether (300 ml), ice-cold water (100 ml), and ice-cold saturated aqueous potassium carbonate (50 ml). The mixture is shaken vigorously and the layers are separated. The aqueous portion is extracted with more cold diethyl ether (2 x 100 ml). The combined ether solution is washed with brine, dried with magnesium sulfate, and evaporated under vacuum to provide dibenzyl (*S*)-aspartate (31.4 g, 0.1 mole) as a colorless liquid.

The dibenzyl (*S*)-aspartate in anhydrous diethyl ether (200 ml) is cooled in an ice-bath under a nitrogen atmosphere. Trimethylchlorosilane (12.7 ml, 0.1 mole) is added to the stirred solution to give a white precipitate. Triethylamine (14.0 ml, 0.1 mole) is then added to the mixture. The cooling bath is removed and the mixture is stirred at room temperature (22—25°C) for 2 hrs. The mixture is then filtered directly into a 3-neck, 1.0 liter, round bottom flask fitted with a sintered glass funnel, magnetic stirrer, and a vacuum-nitrogen inlet. This operation is carried out under a blanket of nitrogen, care being taken to exclude atmospheric moisture. The sintered glass funnel is replaced by a stopper and the ether is evaporated under vacuum with stirring to provide dibenzyl (*S*)-N-trimethylsilylaspartate (35.5 g, 0.092 mole) as a slightly hazy oil.

Anhydrous diethyl ether (250 ml) is added to the flask containing the silyl derivative and the magnetic stirrer is replaced by a mechanical stirrer. The resulting solution is stirred under a nitrogen atmosphere with ice-bath cooling. Ethereal ethyl magnesium bromide (34 ml of a 2.94 M solution, 0.1 mole) is added dropwise over 40 min. to give a cream colored, stirable precipitate. The cooling bath is removed and the mixture is stirred at room temperature. After 1.5 hrs, a viscous gum forms. The mixture is allowed to stand overnight at room temperature. The mixture is then cooled in an ice-methanol bath while ammonium chloride saturated 2N hydrochloric acid (100 ml) is added slowly with stirring. The resulting mixture is diluted with ethyl acetate (100 ml) and water (100 ml) and the layers are separated. The aqueous portion is extracted with more ethyl acetate (3 x 100 ml). The combined organic solution is washed with water (200 ml), 5% aqueous sodium bicarbonate solution (100 ml), water (100 ml), and brine, dried with magnesium sulfate, and filtered. Evaporation of the solvent under vacuum gives an orange oil interspersed with a fine, granular precipitate (25.3 g). This material is dissolved in warm chloroform (75 ml), diluted with petroleum ether (125 ml), seeded, scratched, and cooled in an ice-bath. The precipitate is collected, washed with petroleum ether, and dried under vacuum to give benzyl (*S*)-azetidin-2-one-4-carboxylate (3.85 g) as an off-white solid mp 136—139°C. The mother liquors and washings are combined, diluted with petroleum ether to 500 ml, seeded, and left in a refrigerator for several days. The resulting precipitate is collected, washed with petroleum ether, and dried under vacuum to give additional product (0.82 g) as pale yellow crystals. Recrystallization of a sample from chloroform-petroleum ether gave the product as small, white flakes: mp 141—143°; $[\alpha]_D = -43.4°$ (c3.275 in $CHCl_3$); IR($CHCl_3$) 3425, 1778, 1746 $cm^{-1}$; $^1$H NMR($CDCl_3$) $\delta$ 3.00 (ddd, 1, J = 1.9, 3.2, and 14.6 Hz, H—3a), $\delta$ 3.35 (ddd, J = 1.5, 5.4, and 14.6 Hz, H—3b), $\delta$ 4.20 (dd, 1, J = 3.2 and 5.4 Hz, H—4), $\delta$ 5.22 (s, 2, $OCH_2Ph$), $\delta$ 6.48 (m, 1, NH), 7.38 (s, 5, phenyl); mass spectrum m/e 205 (M+), 163, 91, 70, 43.

| | | | |
|---|---|---|---|
| *Anal.* Calcd. for $C_{11}H_{11}NO_3$: | C, 64.38; | H, 5.40; | N, 6.83. |
| Found: | C, 64.10; | H, 5.70; | N, 6.77. |

*STEP B*

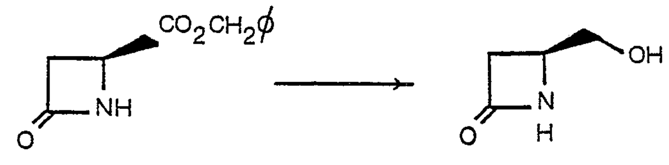

4(*S*)-4-Hydroxymethylazetidin-2-one

Sodium borohydride (3.69 g, 97.5 mmol) is added in one portion to a suspension of benzyl 4(*S*)-azetidin-2-one-4-carboxylate (20.0 g, 97.5 mmol) in 300 ml of absolute methanol at 0°C. The mixture is then allowed to warm slowly with periodic cooling being supplied to keep the internal temperature <30°C. After stirring for 2 hr., glacial acetic acid (23.4 g, 390 mmol) is added and the reaction mixture is concentrated under vacuum. The residue is treated with 500 ml of chloroform and filtered. The filtrate is concentrated under vacuum and the residue is chromatographed on 250 g of silica gel (4:1, chloroform: methanol) to yield 9.62 g (98%) of 4(*S*)-hydroxymethylazetidin-2-one as a white solid: m.p. 51—53°C; $[\alpha]_D = +68.0°$ (C = 2.676 in $CHCl_3$); IR ($CHCl_3$) 3410, 1765 $cm^{-1}$ 1H NMR ($CDCl_3$) $\delta$ 7.07 (1H, br. s, N*H*), $\delta$ 4.05 (1H, br. s, O*H*), $\delta$ 3.77 (2H, m H4, H—5a or b), $\delta$ 3.58 (1H, dd, J = 11, 6, H—5a or b), $\delta$ 2.97 (1H, ddd, J = 14.5, 4.8, 1.3, H3b), $\delta$ 2.7 (1H, br. d, J = 14.5, H3a); mass spectrum m/e 101 (M+), 83.

*STEP C*

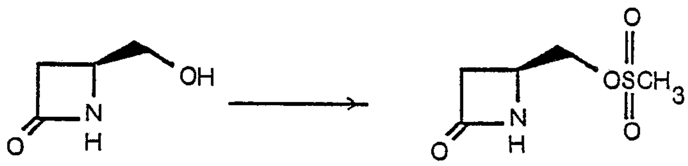

4(S)-4-Methanesulfonyloxymethyl azetidin-2-one

Methane sulfonyl chloride (11.46 g, 100 mmol) is added dropwise by syringe to a solution of 4(*S*)-4-hydroxymethyl azetidin-2-one (10.1 g, 100 mmol) and triethyl amine (10.1 g, 100 mmol) in 15 ml of dry methylene chloride at 0°C. (Warming is necessary in order to initially solubilize the alcohol. The resulting solution is then cooled to 0°C prior to addition of the other reagents). The resulting solution is stirred at 0°C for 1 hr. during which time a voluminous precipitate is produced. At the end of this time, the reaction mixture is filtered and the filtrate is concentrated under vacuum. The two solid residues are combined and treated with 500 ml of chloroform. The resulting mixture is filtered to yield substantially pure 4(*S*)-4-methanesulfonyloxymethyl azetidin-2-one as a white solid. The filtrate, which contains most of the triethylamine hydrochloride, is concentrated under vacuum and chromatographed on 200 g of silica gel (4:1 chloroform:methanol) to yield an additional quantity of mesylate. This material is combined with that obtained previously and recrystallized from chloroform to yield 15.57 g (87%) of 4(*S*)-4-methanesulfonyloxymethylazetidin-2-one as colorless needles: m.p. 109.5—110.5°C; $[\alpha]_D = +25.8°$ (C = 1.025 in $H_2O$); NMR ($D_2O$) $\delta$ 4.62 (1H, dd, J = 11.2, 3.0, H—5a or b), $\delta$ 4.43 (1H, dd, J = 11.2, 6, H—5a or b), $\delta$ 4.12 (1H, m, H4) $\delta$ 3.26 (3H, s

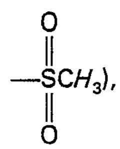

$\delta$ 3.19 (1H, dd, J = 15, 4.5, H3b). $\delta$ 2.88 (1H, dd, J = 15, 2.5, H3a); mass spectrum m/e 179 (M+), 136;

| | | | | |
|---|---|---|---|---|
| *Anal:* Calc: | C, 33.51; | H, 5.06; | N, 7.82; | S, 17.89 |
| Found: | C, 33.54; | H, 5.08; | N, 7.72; | S, 17.93 |

STEP D

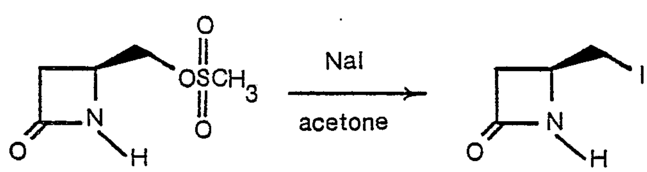


4(S)-4-Iodomethylazetidin-2-one

A mixture of 4(*S*)-4-methanesulfonyloxy azetidin 2-one (11.8 g, 65.9 mmol) and powdered sodium iodide (19.8 g, 132 mmol) in 130 ml of acetone is heated at reflux for 6 hr. The resulting reaction mixture is concentrated *in vacuo,* treated with 200 ml of chloroform and filtered. The filtrate is washed with 2 x 50 ml of water and dried over magnesium sulfate. The organic phase is filtered, concentrated *in vacuo*, and chromatographed on 250 g of silica gel (ethyl acetate) to yield 11.94 g (86%) of 4(*S*)-4-iodomethyl-azetidin-2-one as a white solid. This material is recrystallized from ether-petroleum ether to yield white crystals: mp 91—92°C; $[\alpha]_D = -23.7°$ (C = 1.354 in $CHCl_3$); IR ($CHCl_3$) 3450, 1765 $cm^{-1}$, 1H NMR ($CHCl_3$) $\delta$ 6.13 (brs, N—*H*), $\delta$ 3.94 (m, 1 H, Hc), $\delta$ 3.36 (m, 2H, Hd and e), $\delta$ 3.16 (ddd, 1H, J = 14.9, 5.4, 2.3, Ha), $\delta$ 2.72 (d, d, d, 1H, J = 14.9, 2.1, 2, Hb) mass spectrum m/e 211 ($M^+$), 168, 142, 127, 84.

*STEP E*

Preparation of (4*S*)-1-(t-Butyldimethylsilyl)-4-iodomethylazetidin-2-one

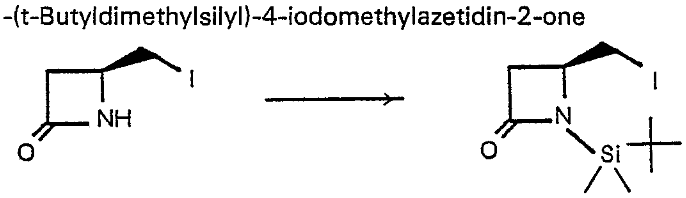

t-butyldimethylchlorosilane (7.51 g, 49.8 mmol) is added in one portion to an ice-cold, stirring solution

of (4*S*)-4-iodomethyl-azetidin-2-one (10.0 g, 47.4 mmol) and triethylamine (5.04 g, 49.8 mmol) in anhydrous dimethylformamide (100 ml). A voluminous white precipitate forms almost immediately. The reaction mixture is stirred at 0—5° for 1 hour and then allowed to warm to room temperature. Most of the solvent is removed under vacuum to give a residue which is partitioned between diethyl ether (250 ml) and water. The ethereal phase is washed with 2.5N hydrochloride acid (50 ml), water (3 x 50 ml), and brine, dried with magnesium sulfate, filtered, and evaporated under vacuum to provide (4*S*)-1-(t-butyldimethylsilyl)-4-iodomethyl-azetidin-2-one (15.1 g) as a white solid. Recrystallization from petroleum ether — ethyl ether gives the product as colorless plates, mp 71—72°; n.m.r. ($CDCl_3$), $\delta$ 3.8 (m,1), $\delta$ 2.6—3.6 (2 overlapping d of AB, 4) $\delta$ 1.0 (S,9), $\delta$ 0.3 (S,6), $\delta$ 0.25 (S,6).

*STEP F*

(4*S*)-1-(t-butyldimethylsilyl)-4-[2,2-(trimethylenedithia)-2-trimethylsilylethyl]-azetidin-2-one

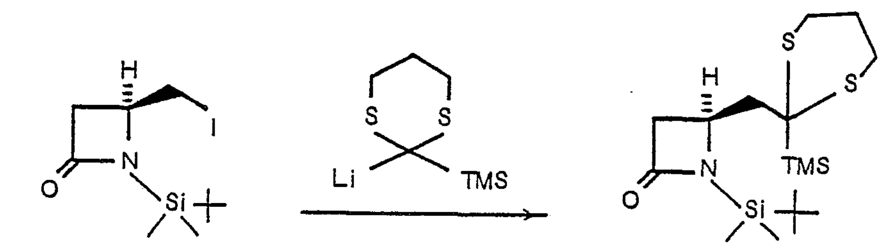

21

A solution of 2-trimethylsilyl-1,3-dithiane (3.78 g, 19.69 mmole) in anhydrous tetrahydrofuran (25 ml) at 0°C is stirred under nitrogen while n-butyllithium in hexane (20.67 mmol) is added dropwise. The resulting solution is stirred for 15 min. at 0°C then cooled to −78°C (dry ice-acetone). A solution of (4*S*)-1-(t-butyldimethylsilyl)-4-iodomethylazetidin-2-one (6.40 g, 19.69 mmol) in 20 ml of anhydrous tetrahydrofuran is added slowly by syringe over ca. 5 min. The resulting solution is stirred at −78°C for 1 hr., then quenched by the addition of saturated aqueous ammonium chloride solution (10 ml) and allowed to warm to room temperature (22°C). The mixture is poured into a separatory funnel containing ethylether (200 ml) and water (100 ml). The organic phase is separated, washed with brine and dried over anhydrous magnesium sulfate. The solvent is removed *in vacuo* to yield a yellow oil. This material is filtered through a short silica gel column (25% ether in petroleum ether) to give 6.15 g (80%) of (4*S*)-1-(t-butyldimethylsilyl)-4-[2,2-(trimethylenedithia)-2-trimethylsilylethyl]-azetidin-2-one as a white solid, m.p. 71—73°C. n.m.r. ($CDCl_3$) $\delta$ 3.9 (1H, m, H—5), 2.2—3.6 (8H, overlapping m), $\delta$ 2.0 (2H, m, $SCH_2CH_2CH_2S$), $\delta$ 0.99 (9H, S, ±Si), $\delta$ 0.23 (15H, br.S, $(CH_3)_2Si$ and $(CH_3)_3Si$). IR ($CHCl_3$) 2930, 2855, 1723 $cm^{-1}$.

Example 2

(3,*R*,*S*,4*R*)-1-(t-Butyldimethylsilyl)-3-[(*R*,*S*)-1-hydroxyethyl]-4-[2,2-(trimethylenedithia)-2-trimethylsilylethyl]-azetidin-2-one (22)

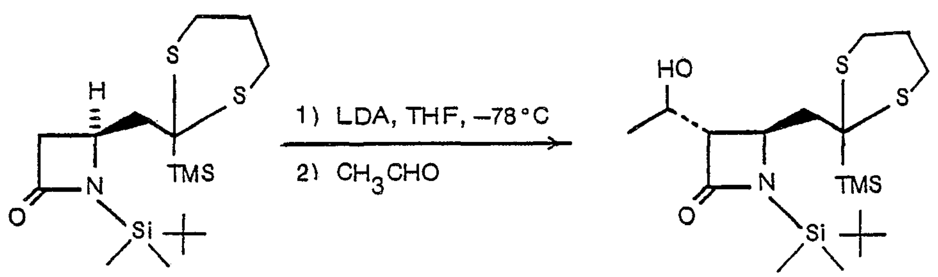


21

22

A solution of diisopropylamine (10.5 mmol) in anhydrous tetrahydrofuran (40 ml) is cooled to −78°C (dry ice-acetone) and stirred under nitrogen atmosphere while n-butyllithium in hexane (10.5 mmol) is added slowly by syringe. After 15 min., a solution of (4*S*)-1-(t-butyldimethylsilyl)-4-[2,2-(trimethylenedithia)-2-trimethylsilyl ethyl]-azetidin-2-one (10.0 mmol) in anhydrous tetrahydrofuran (12 ml) is added slowly by syringe. The resulting solution is stirred at −78°C for 20 min. prior to the addition of acetaldehyde (30.0 mmol). After an additional 10 min. at −78°C the reaction is quenched by the addition of saturated aqueous ammonium chloride solution (10 ml) and allowed to warm to room temperature. The reaction mixture is diluted with ethyl acetate (150 ml) and washed with 2.5N hydrochloric acid solution (50 ml), water (50 ml) and brine (50 ml) and dried over magnesium sulfate. Removal of solvents *in vacuo* gives a white solid (4.6 g) which is chromatographed on 250 g of silica gel (1:1, ether: petroleum ether) to give four main product fractions with a total weight of 4.185 g

(96.7%). Fraction No. 1—$R_f = 0.62$, 85 mg. Fraction No. 2—$R_f = 0.43$, 1.95 g (45%), (3*S*,4*R*)-1-(t-butyldimethyl-silyl)-3-[(S)-1-hydroxyethyl]-4-[2,2-(trimethylenedithia]-2-trimethylsilylethyl]-azetidin-2-one. Fraction No. 3—$R_f = 0.34$, 150 mg. mixture. Fraction No. 4—$R_f = 0.28$, 2.0 g (46%), (3*S*,4*R*)-1-(t-butyldimethylsilyl)-3-[(*R*)-1-hydroxyethyl]-4-[2,2-(trimethylenedithia)-2-trimethylsilylethyl]-azetidin-2-one.

Example 3

(3*S*, 4*R*)-1-(t-Butyldimethylsilyl)-3-(1-oxoethyl)-4-[2,2-(trimethylenedithia)-2-trimethylsilylethyl]azetidin-2-one (22a)

*STEP A*

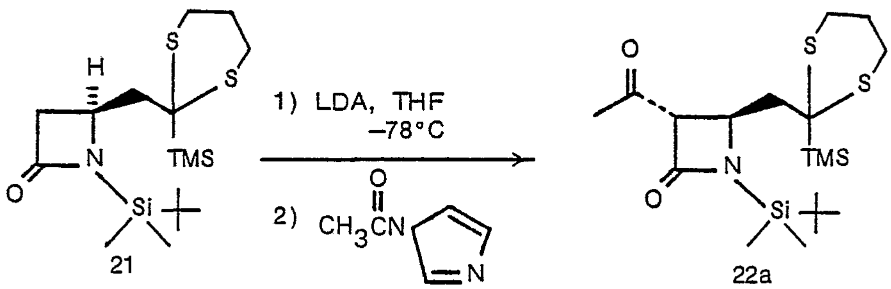


21

22a

A solution of diisopropylamine (6.0 mmol) in anhydrous tetrahydrofuran (25 ml) is cooled to −78°C (dry ice-acetone) and stirred under a nitrogen atmosphere while n-butyllithium in hexane (6.0 mmol) is added by syringe. After 15 min., a solution of (4S)-1-(t-butyldimethylsilyl)-4-[2,2-(trimethylenedithia)-2-trimethylsilylethyl]-azetidin-2-one (3.0 mmol) in anhydrous tetrahydrofuran (3 ml) is added dropwise by syringe. The resulting solution is stirred at −78°C for 30 min., then added through a Teflon tube to a mixture of N-acetylimidazole (6.0 mmol) and anhydrous tetrahydrofuran (25 ml) at −78°C. The resulting yellow reaction mixture is stirred at −78°C for 10 min., then quenched by addition of saturated aqueous ammonium chloride solution. The reaction mixture is poured into ether (200 ml) and extracted with 2.5N hydrochloric acid solution (50 ml), water (50 ml) and brine and dried over magnesium sulfate. Removal of solvents *in vacuo* gives a yellow oil which is chromatographed on silica gel (ether-petroleum ether) (1:2) to yield (3*S*,4R)-1-(t-butyldimethylsilyl)-3-(1-oxoethyl)-4-[2,2-(trimethylenedithia)-2-trimethysilylethyl]-azetidin-2-one.

Example 4

(3S,4R)-1-(t-butyldimethylsilyl)-3-(1-oxoethyl)-4-[2,2-(trimethylenedithia)-2-trimethylsilylethyl)-azetidin-2-one (22a)

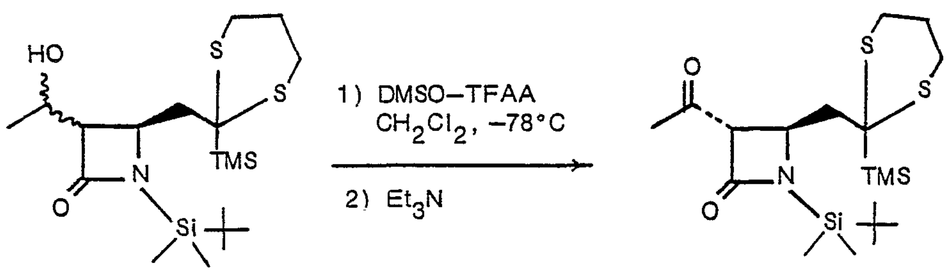


22a

Trifluoroacetic anhydride (6.0 mmol) is added by syringe to a solution of dimethylsulfoxide (8.0 mmol) in anhydrous methylene chloride (10 ml) at −78°C. The resulting mixture is stirred at −78°C for 20 min., during which time a white precipitate forms. A solution of (3*RS*, 4*R*)-1-(t-butyldimethylsilyl)-3-(*RS*-1-hydroxyethyl)-4-[2,2-(trimethylenedithia)-2-trimethylsilylethyl]-azetidin-2-one (4.0 mmol) in anhydrous methylene chloride (10 ml) is added by syringe and the resulting mixture is stirred at −78°C for 40 min. Triethylamine (11.2 mmole) is added by syringe and the cooling bath is removed. After 1 hr. the reaction mixture is diluted with $CH_2Cl_2$ (100 ml) and washed with 2.5N hydrochloric acid solution (50 ml), water (50 ml) and brine and dried over magnesium sulfate. Purification as above yields (3*S*, 4*R*)-1-(t-butyldimethylsilyl)-3-(1-oxoethyl)-4-[2,2-trimethylenedithia)-2-trimethylsilylethyl]-azetidin-2-one. n.m.r. ($CDCl_3$) 4.23 (1H, BrS, H—6), $\delta$ 4.2 (1H, m, H—5), $\delta$ 2.1—3.2 (6H, m), $\delta$ 2.27 (3H, S, $CH_3$—C=O), $\delta$ 2.0 (2H, m, $SCH_2CH_2CH_2S$), $\delta$ 0.96 (9H, S, ±Si), $\delta$ 0.25 (15H, br.S $(CH_3)_2Si$ and $(CH_3)_3Si$).

Example 5

Thienamycin

*STEP A*

(3*S*, 4*R*)-1-(t-Butyldimethylsilyl)-3-[(*R*)-1-hydroxyethyl]-4-[2,2-(trimethylenedithia-2-trimethylsilylethyl]azetidin-2-one (22)

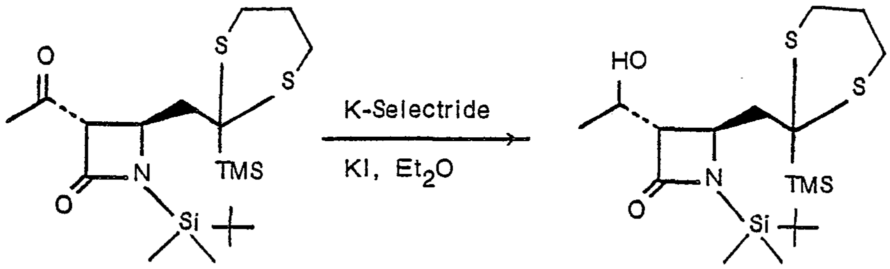


22a 22

K-Selectride (potassium tri-sec-butylborohydride) (4.8 mmol) in a solution of tetrahydrofuran is added dropwise by syringe to a mixture of (3*S*, 4*R*)-1-(t-butyldimethylsilyl)-3-(1-oxoethyl)-4-[2,2-(trimethylenedithia)-2-trimethylsilylethyl]-azetidin-2-one (2.0 mmol) and potassium iodide (2.0 mmol) in anhydrous ether (20 ml) at room temperature. The resulting mixture is stirred at room temperature for 2.5 hr., then quenched by addition of glacial acetic acid (9.6 mmol). The resulting mixture is diluted with ethyl acetate (50 ml) and filtered through celite. The solvents are removed *in vacuo* to give an oil which is chromatographed on silica gel (1:1, ether: petroleum ether) to give (3*S*,4*R*)-1-(t-butyldimethylsilyl)-3-[(*R*)-1-hydroxyethyl]-4-[2,2-(trimethylenedithia)-2-trimethylsilylethyl]-azetidin-2-one as a white solid, n.m.r. ($CDCl_3 + D_2O$) $\delta$ 4.23 (1H, dq, J = 7.5, 7, H—8) $\delta$ 3.78 (1H, ddd, J = 7.5, 3, 2.2, H—5) $\delta$ 3.18 (1H, dd, 7.5, 2.2, H—6), $\delta$ 2.5—3.0 (4H, m, —$SCH_2CH_2CH_2S$— $\delta$ 2.35

(2H, m, 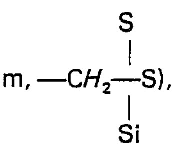),

$\delta$ 2.0 (2H, m, $SCH_2CH_2CH_2S$) $\delta$ 1.33 (3H, d, J = 7, $CH_3$—), $\delta$ 0.98 (9H, S, ±Si) $\delta$ 0.26 (15H, br.S, $(CH_3)_2$ Si + $(CH_3)_3Si$).

*STEP B*

(3*S*,4*R*)-1-(t-Butyldimethylsilyl)-3-[(R)-1-hydroxyethyl]-4-(2-oxo-2-trimethylsilylethyl)-azetidin-2-one (23)

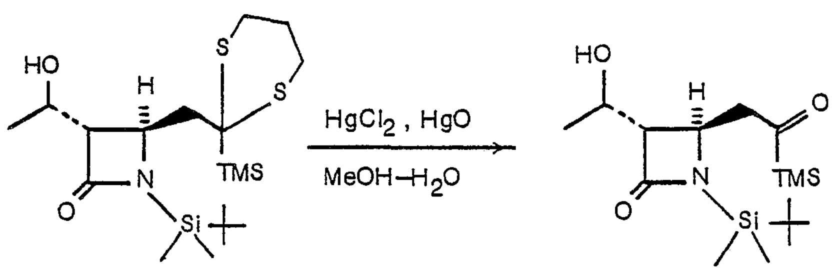


22 23

A mixture of mercuric oxide (6.93 mmol), mercuric chloride (10.2 mmol) and (3*S*,4*R*)-1-(t-butyldimethylsilyl)-3-[(R)-1-hydroxyethyl]-4-[2,2-(trimethylenedithia)-2-trimethylsilylethyl]-azetidin-2-one (4.62 mmol) in 5% aqueous methanol (25 ml) is heated at reflux for 45 min. During this time, the color of reaction mixture changes from orange to off-white. The mixture is cooled and filtered and the filter cake is washed several times with methanol. The combined filtrate and washings are concentrated to ~5 ml *in vacuo*, then diluted with ethyl acetate (100 ml) and washed with saturated aqueous ammonium chloride solution (2 x 50 ml) and brine. The organic phase is dried over magnesium sulfate and concentrated *in vacuo* to yield a pale yellow oil. This material is chromatographed on silica gel (ether) to yield (3*S*, 4*R*)-1-(t-butyldimethylsilyl)-3-[(*R*)-1-hydroxyethyl]-4-(2-oxo-2-trimethylsilylethyl)-azetidin-2-one, 1.38 g (87%), as a white solid, m.p. 82—84°C. n.m.r. ($CDCl_3$—$D_2O$) $\delta$ 3.6—4.3 (2H, m, H—5, H—8) $\delta$ 3.12 (2H, center of d of AB, J = 18, 4, 8.5,

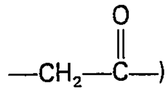)

δ 2.7 (1H, dd, J = 7.5, 2, H—6), δ 1.27 (3H, d, J = 6.5, $CH_3$—) δ 0.99 (9H, s, ±Si), δ 0.3 (15H, br.S, $(CH_3)_3$ Si and $(CH_3)_2$Si). I.R. ($CHCl_3$) 3450, 2930, 2855, 1737, 1635 $cm^{-1}$.

*STEP C*

(3*S*,4*R*)-1-(t-Butyldimethylsilyl)-3-[(*R*)-1-hydroxyethyl]-4-carboxymethyl-azetidin-2-one (24)

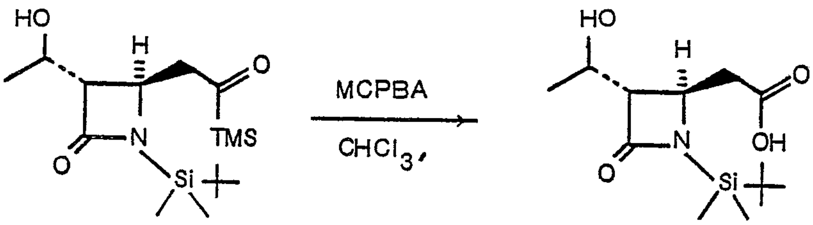


23 24

m-Chloroperbenzoic acid (1.00 mmol) is added to a solution of (3*S*,4*R*)-1-(t-butyldimethylsilyl-3-[(*R*)-1-hydroxyethyl]-4-(2-oxo-2-trimethylsilylethyl)-azetidin-2-one (1.00 mmol) in chloroform (4 ml). The resulting solution is heated at reflux for 4 hr, then cooled, concentrated *in vacuo*, and the residue chromatographed on silica gel (2% glacial acetic acid in methylene chloride). (3S, 4R)-1-(t-butyldimethylsilyl)-3-[(R)-1-hydroxyethyl]-4-carboxymethyl-azetidin-2-one, 238 mg (83%) is isolated as a colorless solid, $R_f = 0.25$. n.m.r. ($CDCl_3$ and $D_2O$) δ 3.6—4.3 (2H, m, H—5, H—8), δ 2.98 (1H, dd, J = 7, 2.1 H—6), δ 2.7 (2H,d of ABq)—$CH_2CO_2H$), δ 1.29 (3H, d, J = 6, $CH_3$—) δ 0.95 (9H, S, Si±), δ 0.25 (6H, S, $(CH_3)_2$—Si).

*STEP D*

(3*S*, 4*R*)-1-(t-Butyldimethylsilyl)-3-[(*R*)-1-hydroxyethyl]-4-(3-p-nitrobenzyloxycarbonyl-2-oxopropyl)-azetidin-2-one (25)

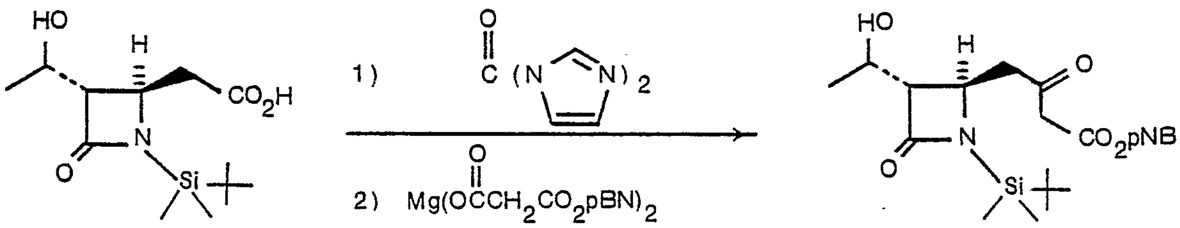


24 25

1,1′-Carbonyldimidazole (1.10 mmol) is added in one portion to a solution of (3*S*, 4*R*)-1-(t-butyldimethylsilyl-3-[(*R*)-1-hydroxyethyl]-4-carboxymethyl-azetidin-2-one (1.0 mmol) in anhydrous tetrahydrofuran (5 ml) at room temperature. The resulting solution is stirred at room temperature for 6 hours. In a second flask, magnesium ethoxide (5 mmol) is added in one portion to a solution of the mono-p-nitrobenzyl ester of malonic acid (10 mmol) in anhydrous tetrahydrofuran (25 ml). The resulting mixture is stirred at room temperature for 1 hr, then the tetrahydrofuran is removed at the pump and the gummy residue is triturated with ether to yield the magnesium salt as an off-white solid. (1.1 mmol) of this magnesium salt is then added to the first reaction flask and the resulting mixture is stirred at room temperature for 18 hrs. The reaction mixture is then poured into 50 ml of ether, washed with 0.5N hydrochloric acid solution (20 ml), water (20 ml), saturated aqueous sodium bicarbonate solution (20 ml), brine and dried over magnesium sulfate. Removal of solvents *in vacuo* gives an oil which is chromatographed on silica gel (ether) to yield (3*S*,4*R*)-1-(t-butyldimethylsilyl)-3-[(*R*)-1-hydroxyethyl]-4-(3-p-nitrobenzyloxycarbonyl-2-oxopropyl)-azetidin-2-one. n.m.r. ($CDCl_3$—$H_2O$) δ 8.24, 8.10, 7.52, 7.38 (2H, AB, aromatic), δ 5.26 (2H, S, —$CH_2$—Ar), δ 3.5—4.2 (2H, m, H—5, H—8), δ 2.6—3.3

(3H, m, H—6, $-CH_2-\overset{O}{\overset{\|}{C}}-$),

δ 1.3 (3H, d, J = 6.6, $CH_3$—) δ 0.98 (9H, S, ±Si—) δ 0.25

(6H, S, $(CH_3)_2Si<$ ).

*STEP E*

(3*S*,4*R*)-3-[(*R*)-1-hydroxyethyl]-4-(3-p-nitrobenzyloxycarbonyl-2-oxopropyl)azetidin-2-one (16)

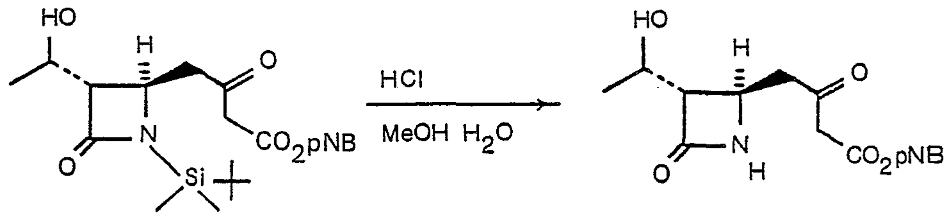


25 16

A solution of (3*S*, 4*R*)-1-(t-butyldimethylsilyl)-3-[(*R*)-1-hydroxyethyl]-4-(3-p-nitrobenzyloxycarbonyl-2-oxopropyl)-azetidin-2-one (1.0 mmol) in 20 ml of 9:1 (v/v) methanol-water is cooled to 0°C. Concentrated hydrochloric acid (0.34 ml) is added and the resulting solution is stirred at 0°C for 15 min., then allowed to warm to room temperature. After 2.5 hrs, at room temperature the reaction mixture is diluted with ethyl acetate (25 ml), washed with water (10 ml) and brine, dried over magnesium sulfate and concentrated *in vacuo* to yield (3*S*, 4*R*)-3-[(*R*)-1-hydroxyethyl]-4-(3-p-nitrobenzyloxycarbonyl-2-oxopropyl)-azetidin-2-one.

*STEP F*

Preparation of (3*S*,4*R*)-3-[(R)-1-hydroxyethyl)-4-[3-(4-nitrobenzyl)oxycarbonyl-2-oxo-3-diazopropyl]azetidin-2-one (17)

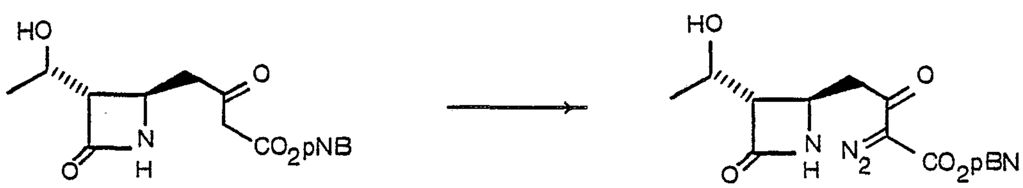

16 17

Triethylamine (263 mg, 2.6 mmol) is added by syringe to a mixture of (3*S*,4*R*)-3-[(R)-1-hydroxyethyl]-4-[3-(4-nitrobenzyl)oxycarbonyl-2-oxopropyl]azetidin-2-one (253 mg, 0.72 mmol) and p-carboxybenzene and sulfonylazide (196 mg, 0.84 mmol) in dry acetonitrile (6 ml) at 0°C. When addition is complete the cooling bath is removed and the reaction mixture is stirred at room temperature for 1 hour. The mixture is then diluted with ethyl acetate (50 ml) and filtered. The filtrate is concentrated *in vacuo* and the residue is chromatographed on a short silica gel column (ethyl acetate) to yield 222 mg. (81% overall from (3*S*, 4*R*)-1-(t-butyldimethylsilyl)-3-[(R)-1-(t-butyl dimethylsilyloxy)ethyl]-4-[3-(4-nitrobenzyl)oxycarbonyl-2-oxopropyl]azetidin-2-one) of (3*S*,4*R*)-3-(R)-1-hydroxyethyl)-4-[3-(4-nitrobenzyl)oxycarbonyl-2-oxo-3-diazopropyl]azetidin-2-one as a white solid m.p. (dec.) 163°C. IR($CHCl_3$, $CM^{-1}$) 3410, 2132, 1756, 1718, 1650, 1350, 1280, 1120; n.m.r. ($CDCl_3$) $\delta$ 7.9 (2d-aromatic, 4), $\delta$ 5.4 (s, 2), 6.2 (brs, 1), $\delta$ 4.1 (m, 2), $\delta$ 4.1 (m, 2), $\delta$ 2.6—3.6 (m, 4), $\delta$ 1.32 (d, 3, J = 6.2).

*STEP G*

Preparation of (5*R*,6*S*)p-Nitrobenzyl 6-[(*R*)1-hydroxyethyl]-1-azabicyclo[3.2.0]heptan-3,7-dione-2-carboxylate (18)

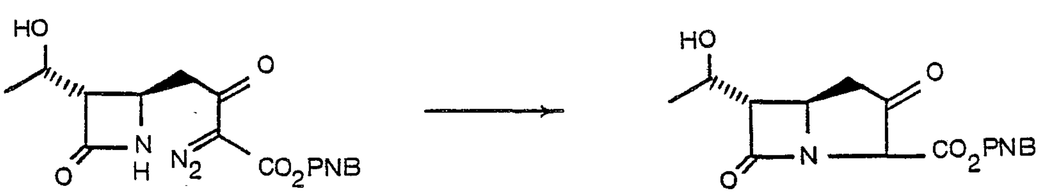

17 18

A suspension of (3*S*,4*R*)-3-[(R)-1-hydroxyethyl]-4-[3-(4-nitrobenzyl)oxycarbonyl-2-oxo-3-diazopropyl]azetidin-2-one (56.4 mg, 0.15 mmol) and rhodium (II) acetate (0.1 mg) in dry benzene (3 ml) is deoxygenated by bubbling through nitrogen for 10 minutes. The mixture is then heated to 78°C for 1 hour. During heating the solid starting material gradually goes into solution. The mixture is then cooled, filtered to remove the catalyst, and the filtrate is concentrated *in vacuo* to yield (5*R*, 6*S*) p-nitrobenzyl

6-[(R)-1-hydroxyethyl]-1-azabicyclo[3.2.0]heptan-3,7-dione-2-carboxylate, 51 mg. (98%) as a colorless oil which slowly crystallized at room temperature (22°C).

Physical Properties:

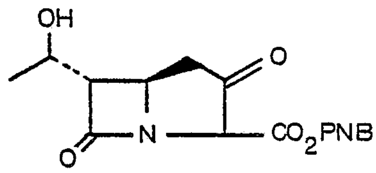

PNB = p-nitrobenzyl

n.m.r.: (300 MHz, $CDCl_3$) $\delta$ 8.26, 7.54 (aromatic, 4), 5.29 (AB, 2), 4.77 (s, 1), 4.32 (dg, I, J = 6.6, 7), 4.16 (ddd, 1, J = 7, 7.5, 2.2), 3.21 (dd, 1, J = 7, 2.2), 2.94 (dd, 1, J = 19.5, 7) 2.50 (dd, 1, J = 19.5, 7.5), 2.2 (brs, 1), 1.37 (d, 3, J = 6.6). I.R.: ($CHCl_3$, $CM^{-1}$) 1770, 1758, 1610, 1522, 1353 m.p. 110—111°C.

*STEP H*

Preparation of *p*-Nitrobenzyloxycarbonylaminoethanethiol

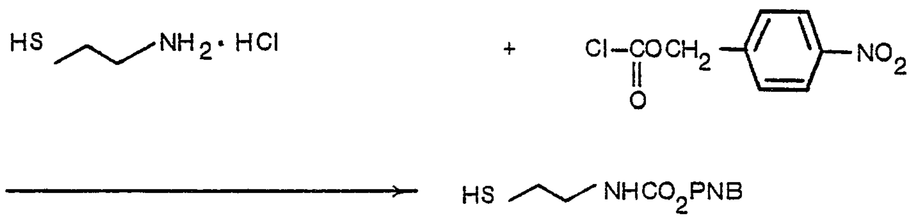

To 600 ml diethyl ether ($Et_2O$ — 75 ml $H_2O$ in an ice bath with stirring is added 3.2 g cysteamine hydrochloride (mw = 114; 28.1 mmole). A solution of 7.14 g $NaHCO_3$ (mw = 84; 85 mmole) in 75 ml $H_2O$ is added. The ice bath is removed, and at room temperature a solution of 6.75 g *p*-nitrobenzylchloroformate (mw = 216; 31.3 mmole) in 270 ml $Et_2O$ is added dropwise over a period of one hour. After 10 additional minutes, the layers are separated. The ether layer is extracted with 150 ml 0.25N HCl, and then with 200 ml brine. Each aqueous layer is then backwashed successively with 100 ml $Et_2O$. The combined $Et_2O$ layers are dried over anhydrous $MgSO_4$, filtered, and concentrated under a $N_2$ stream. The crystalline residue is slurried in a small amount of ether, filtered, and the pale yellow crystals are dried under high vacuum to give 4.7 g. *p*-nitrobenzyloxycarbonylaminoethanethiol (65% yield). NMR ($CDCl_3$): 8.18 (*d*, J = 8 Hz, aromatic protons ortho to nitro), 7.47 (*d*, J = 8 Hz, aromatic protons meta to nitro), 5.27 (—N*H*—), 5.20 (*s*, $CH_2$—NH—), 2.67 (*m*, —$CH_2$—SH), 1.35 (*t*, J = 8.5 Hz, —S*H*) in ppm downfield from TMS. IR ($CHCl_3$ solution): carbonyl — 1725 $cm^{-1}$. M.S.: molecular ion — 256, (M—47) at 209, (M—136) at 120, $^{+}CH_2\emptyset pNO_2$ at 136.

*STEP H2*

Preparation of (5*R*,6*S*) *p*-Nitrobenzyl 3-[2-(p-nitrobenzyloxycarbonyl)amino ethylthio]-6-[(R)-1-hydroxyethyl]-1-azabicyclo[3,2,0]-hept-2-en-7-one-2-carboxylate (20)

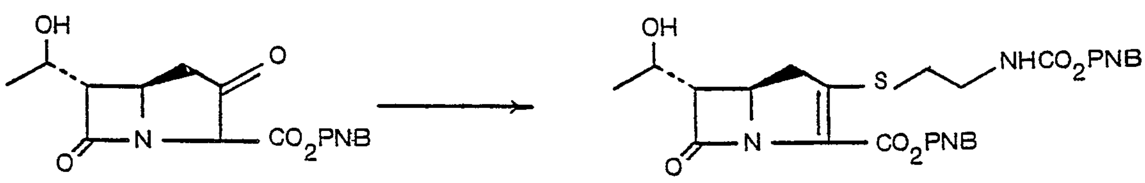

18 20

(5*R*,5*S*) p-Nitrobenzyl 6-[(*R*)1-hydroxyethyl]-1-azabicyclo[3,2,0]heptan-3,7-dione-2-carboxylate (51 mg, 0.147 mmol) is dissolved in acetonitrile (3 ml) and the resulting solution is cooled to 0°C. Diisopropylethylamine (22 mg, 0.17 mmol) is added by syringe and the resulting solution is stirred at 0°C for 1 minute prior to the addition of a solution of freshly recrystallized p-toluene sulfonic anhydride (51 mg., 0.156 mmol) in dry acetonitrile (1 ml). The resulting solution is stirred at 0°C for 1 hour to provide (5*R*,6*S*)p-nitrobenzyl 3-(p-toluenesulfonyloxy)-6-[(R)-1-hydroxyethyl]-1-azabicyclo[3.2.0]hept-2-en-7-one-2-carboxylate, then cooled to −25°C.

Diisopropylethylamine (80.5 mg, 0.624 mmol) is added by syringe followed shortly thereafter by a solution of N-p-nitrobenzyloxycarbonylcysteamine (Step H1; 40 mg, 0.156 mmol) in 1 ml of dry

acetonitrile. The reaction mixture is then stored in a refrigerator for 70 hr. The mixture is diluted with 25 ml of ethyl acetate washed with brine and dried over magnesium sulfate. Solvents are removed *in vacuo* to yield a yellow oil which is chromatographed on a silica gel plate (ethyl acetate, $R_f = 0.4$) to yield (5*R*, 6*S*) p-nitrobenzyl-3-[2-(p-nitrobenzyloxycarbonyl)amino ethylthio]-6-[(R)-1-hydroxyethyl]-1-azabicyclo [3,2,0]-hept-2-en-7-dione-2-carboxylate as a yellow solid, m.p. 167—169°C. IR (Nujol mull) 1773 and 1690 $cm^{-1}$; n.m.r. ($CDCl_3$) $\delta$ 7.54—8.26 (overlapping ABq, 4), $\delta$ 5.40 (ABq, 2), $\delta$ 5.22 (s, 2), $\delta$ 4.27 (m, 2), $\delta$ 3.47 (m), $\delta$ 3.23 (dd, 1), $\delta$ 3.14 (dd, 1), $\delta$ 3.40 (dd, 1), $\delta$ 3.04 (m, 2), $\delta$ 1.37 (d, 3).

*STEP I*

Preparation of Thienamycin (I)

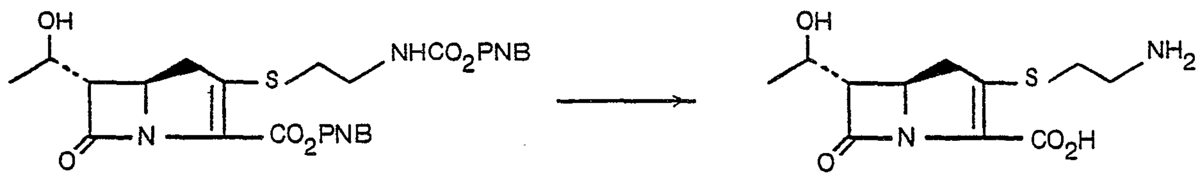

20 I

A mixture of N-p-nitrobenzyloxycarbonyl thienamycin p-nitrobenzyl ester (10 mg, 0.017 mmol) and 10% Pd/C-Bolhofer type in tetrahydrofuran (2 ml), 0.1 M dipotassium hydrogen phosphate solution (1.4 ml) and 2-propanol (0.2 ml) is hydrogenated at 40 psi on the Parr shaker for 30 minutes. The mixture is then filtered and the catalyst is washed with water (3 x 3 ml). The combined filtrate and washings are extracted with ethyl acetate-ethyl ether then concentrated to ~3 ml and lyophilized. The resulting white powder is identical to natural thienamycin in all respects.

**Claims for the Contracting States: BE CH DE FR GB IT LI LU NL SE**

1. A compound selected from the group consisting of:

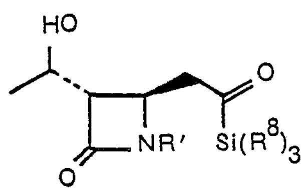

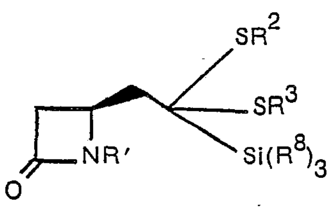

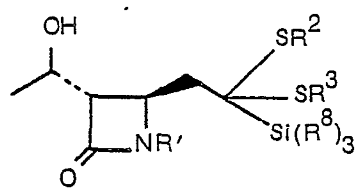

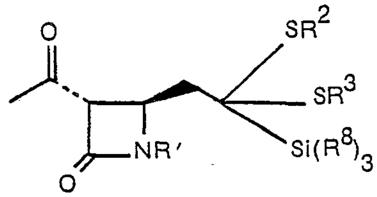

wherein $R^2$ and $R^3$ are independently selected from: lower alkyl having 1—6 carbon atoms, phenyl, benzyl, methoxybenzyl, or trityl; R′ is a triorgano silyl group, 3,4-dimethoxybenzyl or hydrogen; $R^8$ is lower alkyl having 1—6 carbon atoms, or phenyl, or $R^2$ and $R^3$ together form $-(CH_2)_3-$.

2. A compound according to Claim 1, wherein: R′ is triloweralkyl silyl having from 3—12 carbon atoms; $R^8$ is alkyl having 1—3 carbon atoms, or phenyl; and $R^2$ and $R^3$ are: $-(CH_2)_3-$ or alkyl having 1—3 carbon atoms each.

3. A process for preparing

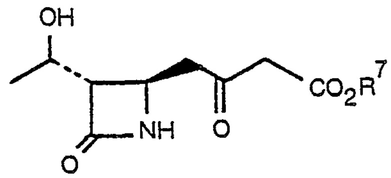

comprising:
oxidizing:

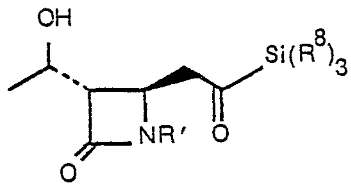

to form:

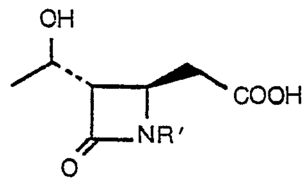

followed by treating with $R^7O_2CCH_2CO_2^{\ominus}$ to yield:

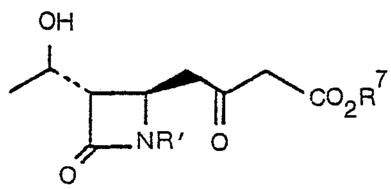

wherein $R^7$ is a carboxyl protecting group and R′ is a triorgano silyl group or 3,4-dimethoxybenzyl and removal of the protecting group R′.

4. A process for preparing

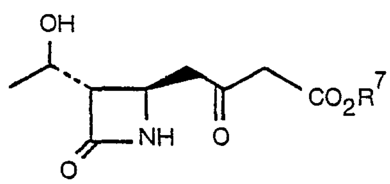

comprising:
oxidizing:

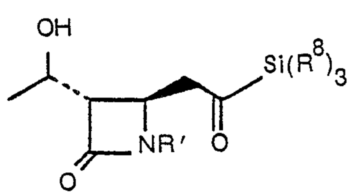

to form:

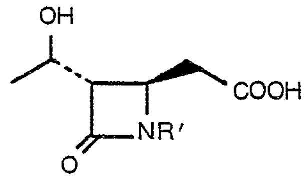

followed by treating with $(R^7O_2CCH_2CO_2)_2Mg$ to yield

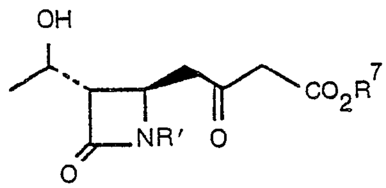

wherein $R^7$ and R′ are defined as in Claim 3 and removal of the protecting group R′.

5. A compound having the structure:

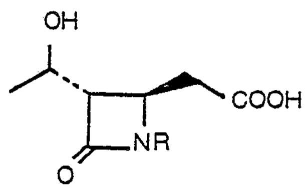

wherein R is a triorgano silyl group or 3,4-dimethoxybenzyl.

6. A compound according to Claim 5 wherein: R is trialkylsilyl wherein the alkyl moieties have 1—6 carbon atoms each.

7. A compound according to Claim 6 wherein R is (*t*-butyl)dimethylsilyl.

**Claims for the Contracting State: AT**

1. A process for preparing

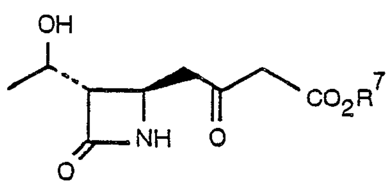

comprising:
oxidizing:

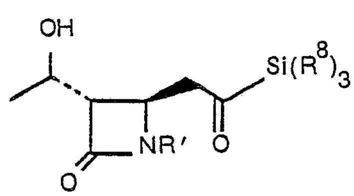

to form:

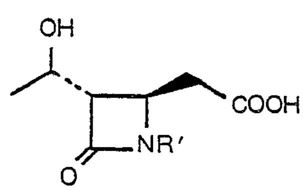

followed by treating with $R^7O_2CCH_2CO_2^{\ominus}$ to yield:

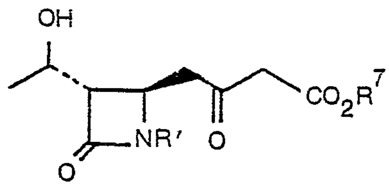

wherein $R^7$ is a carboxyl protecting group and R′ is a triorgano silyl group or 3,4-dimethoxybenzyl and removal of the protecting group R′.

2. A process for preparing

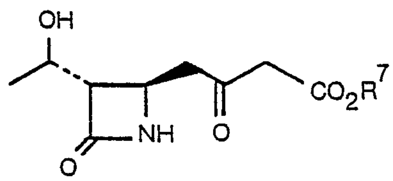

comprising:
oxidizing:

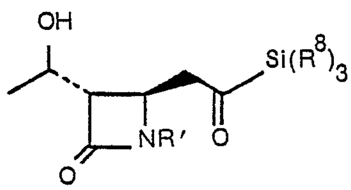

to form:

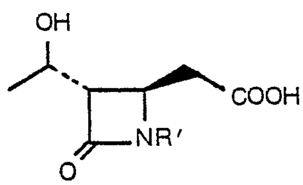

followed by treating with $(R^7O_2CCH_2CO_2)_2Mg$ to yield

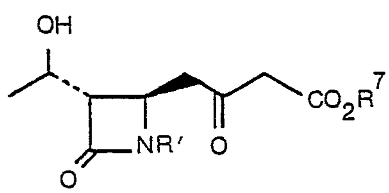

wherein $R^7$ and R′ are defined as in Claim 1 and removal of the protecting group R′.

**Revendications pour les Etats contractants: BE CH FR GB IT LI LU NL SE DE**

1. Un composé choisi dans le groupe constitué par:

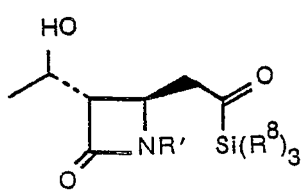

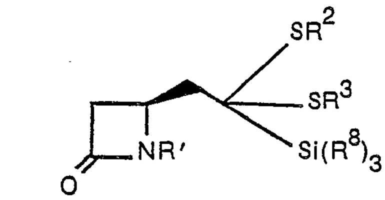

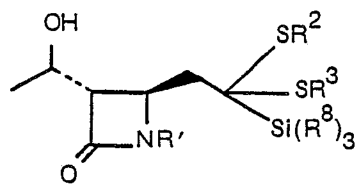

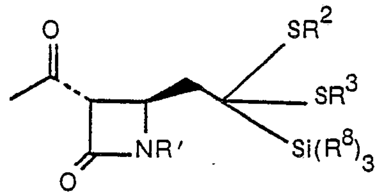

où $R^2$ et $R^3$ sont choisis indépendamment parmi: un allyle inférieur ayant 1 à 6 atomes de carbone, un phényle, un benzyle, un méthoxybenzyle ou un trityle; R′ est un groupe triorganosilyle, un 3,4-diméthoxybenzyle ou un hydrogène; et $R^8$ est un alkyle inférieur ayant 1 à 6 atomes de carbone ou un phényle, ou $R^2$ et $R^3$ forment ensemble $-(CH_2)_3-$.

2. Un composé selon la revendication 1, dans lequel: R′ est un trialkyl(inférieur)silyle ayant 3 à 12 atomes de carbone; $R^8$ est un alkyle ayant 1 à 3 atomes de carbone ou un phényle; et $R^2$ et $R^3$ sont: $-(CH_2)_3-$ ou chacun un alkyle ayant 1 à 3 atomes de carbone.

3. Un procédé pour préparer

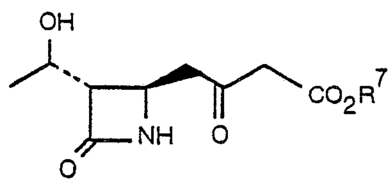

comprenant:
l'oxydation de:

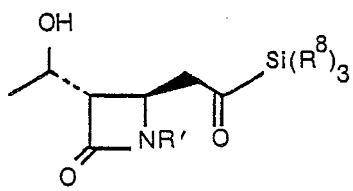

pour former:

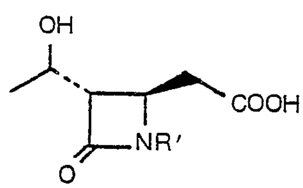

puis le traitement avec $R^7O_2CCH_2CO_2^{\ominus}$ pour fournir:

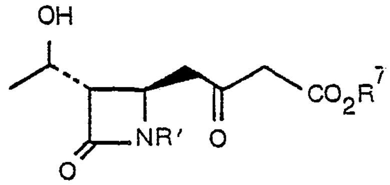

où $R^7$ est un groupe carboxy-protecteur, et R′ est un groupe triorganosilyle ou un 3,4-diméthoxybenzyle, et l'élimination du groupe protecteur R′.

4. Un procédé pour préparer

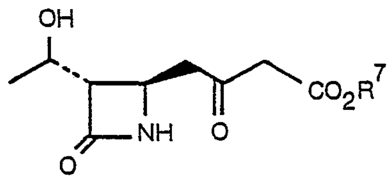

comprenant:
l'oxydation de:

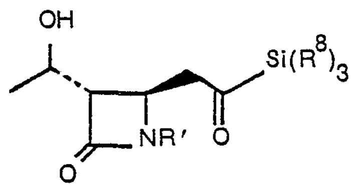

pour former:

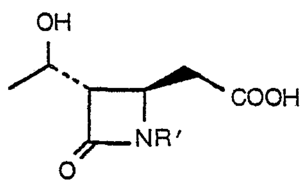

puis le traitement avec $(R^7O_2CCH_2CO_2)_2Mg$ pour fournir:

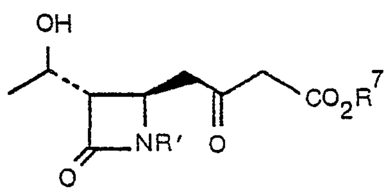

où $R^7$ et R′ sont comme défini dans la revendication 3, et l'élimination du groupe protecteur R′.

5. Un composé ayant pour structure:

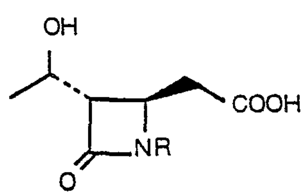

dans laquelle R est un groupe triorganosilyle ou un 3,4-diméthoxybenzyle.

6. Un composé selon la revendication 5, où: R est un trialkylsilyle dont les fragments alkyles ont chacun 1 à 6 atomes de carbone.

7. Un composé selon la revendication 6, dans lequel R est un (tert-butyl)diméthylsilyle.

**Revendications pour l'Etat contractant: AT**

1. Un procédé pour préparer

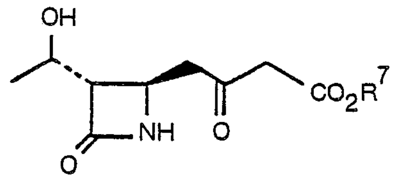

comprenant:
l'oxydation de:

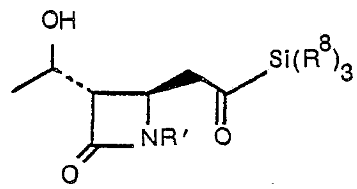

pour former:

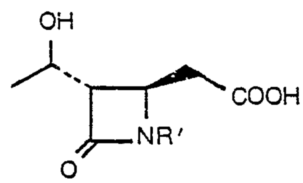

puis le traitement avec $R^7O_2CCH_2CO_2^{\ominus}$ pour fournir:

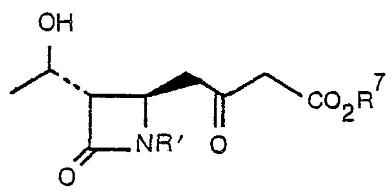

où $R^7$ est un groupe carboxy-protecteur et R' est un groupe triorganosilyle ou un 3,4-diméthoxybenzyle, et l'élimination du groupe protecteur R'.

2. Un procédé pour préparer

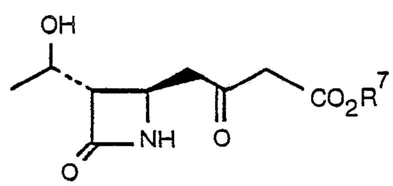

comprenant:
l'oxydation de:

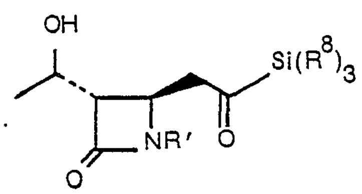

pour former:

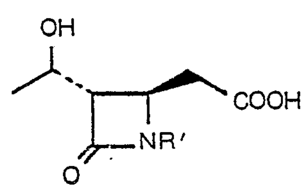

puis le traitement avec $(R^7O_2CCH_2CO_2)_2Mg$ pour fournir:

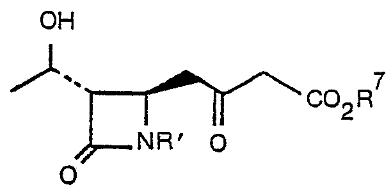

où $R^7$ et R' sont comme défini dans la revendication 1, et l'élimination du groupe protecteur R'.

**Patentansprüche für die Vertragsstaaten: BE CH DE FR GB IT LI LU NL SE**

1. Eine Verbindung, ausgewählt aus der aus:

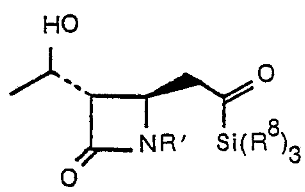

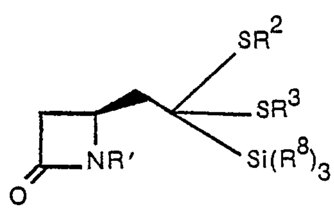

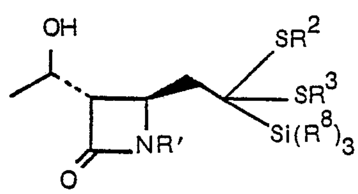

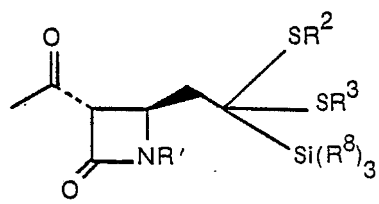

bestehenden Gruppe, worin $R^2$ und $R^3$ unabhängig voneinander aus Niedrigalkyl mit 1—6 Kohlenstoffatomen, Phenyl, Benzyl, Methoxybenzyl oder Trityl ausgewählt sind; R' eine Triorganosilylgruppe, 3,4-Dimethoxybenzyl oder Wasserstoff ist; $R^8$ Niedrigalkyl mit 1—6 Kohlenstoffatomen oder Phenyl ist, oder $R^2$ und $R^3$ zusammengenommen $-(CH_2)_3-$ bilden.

2. Eine Verbindung gemäß Anspruch 1, worin: R' Triniedrigalkylsilyl mit 3—12 Kohlenstoffatomen ist; $R^8$ Alkyl mit 1—3 Kohlenstoffatomen oder Phenyl ist; und $R^2$ und $R^3$ $-(CH_2)_3-$ oder Alkyl mit je 1—3 Kohlenstoffatomen sind.

3. Ein Verfahren zur Herstellung von

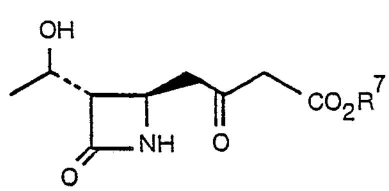

umfassend: das Oxidieren von:

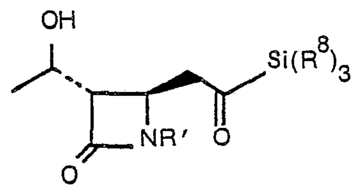

unter Bildung von:

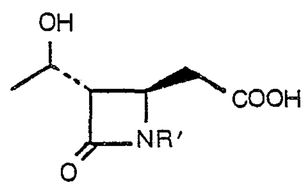

gefolgt von der Behandlung mit $R^7O_2CCH_2CO_2^\ominus$ unter Bildung von:

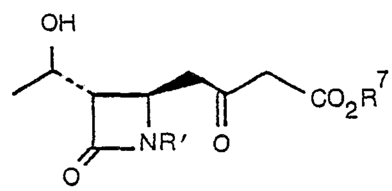

worin $R^7$ eine Carboxylschutzgruppe ist; und R′ eine Triorganosilylgruppe oder 3,4-Dimethoxybenzyl ist, und Entfernung der Schutzgruppe R′.

4. Ein Verfahren zur Herstellung von

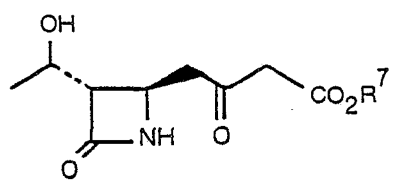

umfassend: das Oxidieren von:

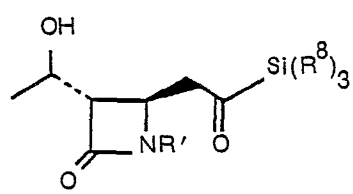

unter Bildung von:

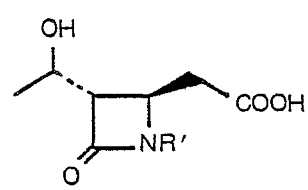

gefolgt von der Behandlung mit $(R^7O_2CCH_2CO_2)_2Mg$ unter Bildung von

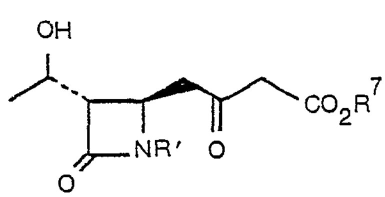

worin $R^7$ und R′ wie in Anspruch 3 definiert sind, und Entfernung der Schutzgruppe R′.

5. Eine Verbindung mit der Struktur:

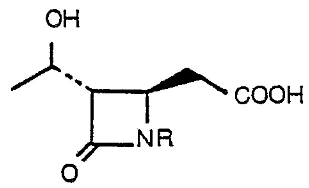

worin R eine Triorganosilylgruppe oder 3,4-Dimethoxybenzyl ist.

6. Eine Verbindung gemäß Anspruch 5, worin: R Trialkylsilyl ist, worin die Alkylreste je 1—6 Kohlenstoffatome haben.

7. Eine Verbindung gemäß Anspruch 6, worin R (tert.Butyl)-dimethylsilyl ist.

**Patentansprüche für den Vertragsstaat: AT**

1. Ein Verfahren zur Herstellung von

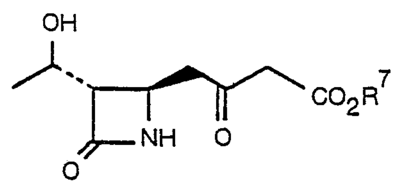

umfassend: das Oxidieren von:

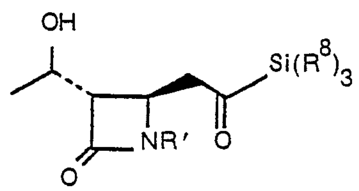

unter Bildung von:

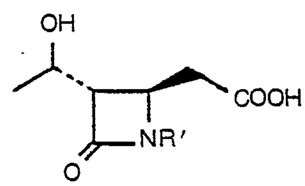

gefolgt von der Behandlung mit $R^7O_2CCH_2CO_2^{\ominus}$ unter Bildung von:

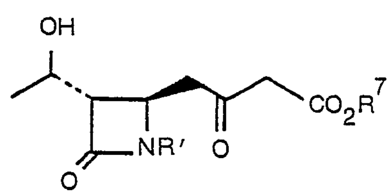

worin $R^7$ eine Carboxylschutzgruppe ist; und R′ eine Triorganosilylgruppe oder 3,4-Dimethoxybenzyl ist, und Entfernung der Schutzgruppe R′.

2. Ein Verfahren zur Herstellung von

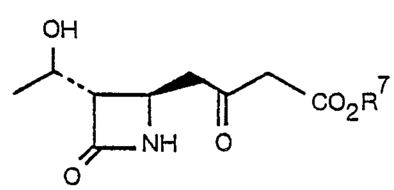

umfassend: das Oxidieren von:

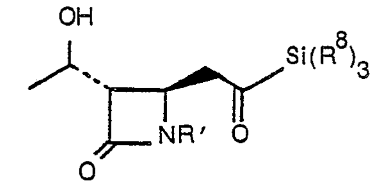

unter Bildung von:

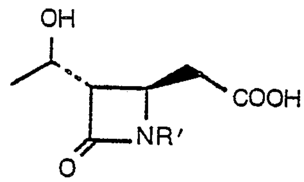

gefolgt von der Behandlung mit $(R^7O_2CCH_2CO_2)_2Mg$ unter Bildung von

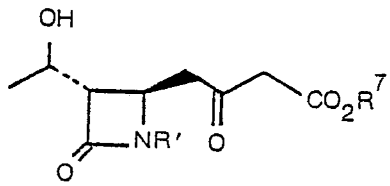

worin $R^7$ und R′ wie in Anspruch 3 definiert sind, und Entfernung der Schutzgruppe R′.